(19) Europäisches Patentamt
European Patent Office
Office européen des brevets

(11) EP 1 867 987 A2

(12) EUROPEAN PATENT APPLICATION

(43) Date of publication:
19.12.2007 Bulletin 2007/51

(51) Int Cl.:
*G01N 33/497* (2006.01)

(21) Application number: 06027079.0

(22) Date of filing: 29.12.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 13.06.2006 US 812989 P

(71) Applicant: ATKINSON VENTURE HOLDINGS LTD.
Calgary, Alberta T2P 4V5 (CA)

(72) Inventor: Pfeiffer, Nicholas
RR3, Mission
British Columbia, V2V 4J1 (CA)

(74) Representative: Hirsz, Christopher Stanislaw et al
HLBBshaw
Merlin House
Falconry Court
Baker's Lane
Epping Essex CM16 5DQ (GB)

(54) **Method and apparatus for determining alcohol content in a breath sample**

(57) A method and apparatus for determining alcohol content in a breath sample, comprising at least one of breath sample exhaust means, resistance lowering means, and correction factor calculation means.

FIG. 2a

EP 1 867 987 A2

Description

FIELD OF THE INVENTION

The present invention relates to alcohol detection methods and apparatus, and more particularly to such detection methods employing fuel cell sensors.

BACKGROUND OF THE INVENTION

A variety of alcohol detection techniques and devices are known in the art, including devices for determining blood alcohol content (BAC) from a breath sample. For example, police officers utilize mobile breath testing devices that employ fuel cell technology to determine if an individual is inebriated and therefore unable to safely operate a motor vehicle.

A micro fuel cell sensor is commonly used to determine the amount of alcohol (ethanol) in the breath sample, and this amount can then be correlated with the amount of alcohol in the blood by known methods. A fuel cell sensor is an electrochemical device in which the substance of interest, such as alcohol, undergoes a chemical oxidation reaction at a catalytic electrode surface (for example, platinum) to generate a quantitative electrical response. By careful electrode design and catalyst selection, the fuel cell chemistry can be geared to work only with a limited range of fuel substances. This high level of analytical specificity is one of the positive features of the fuel cell sensors. Platinum electrochemical fuel cells are recommended as the analytical sensor in instruments intended for both screening and evidential testing applications.

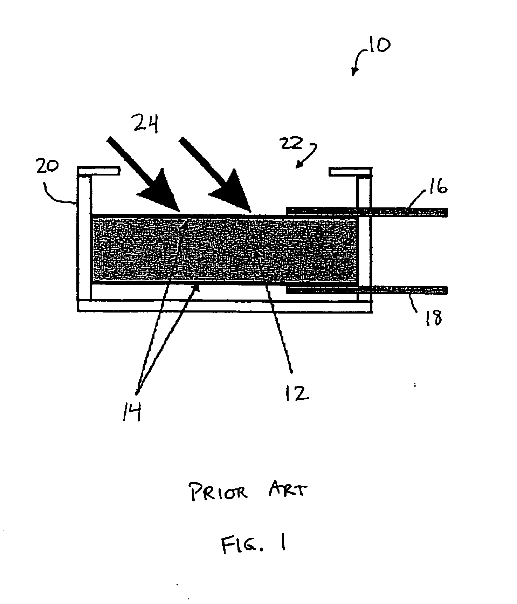

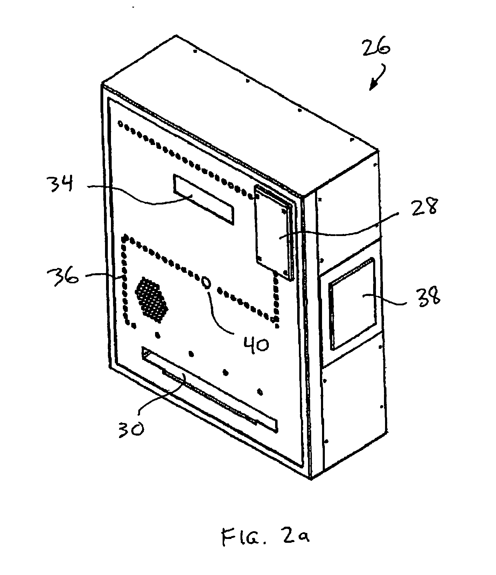

In its simplest form, illustrated in Figure 1, the alcohol fuel cell 10 consists of a porous, chemically inert layer 12 coated on both sides with finely divided platinum (called platinum black) 14. The manufacturer impregnates the porous layer 12 with an acidic electrolyte solution, and applies platinum wire electrical connections 16, 18 to the platinum black surfaces 14 (connection 16 being the positive lead and connection 18 being the negative lead). The manufacturer mounts the entire assembly 10 in a plastic case 20, which also includes a gas inlet 22 that allows a breath sample 24 to be introduced.

The benefit of fuel cell sensors is that the amount of electrical current generated is proportional to the amount of alcohol (ethanol) that is catalyzed at the surface of the fuel cell membrane. The disadvantage, however, is that the fuel cell can quickly saturate so that there is poor correlation between the concentration of alcohol in the breath sample near the fuel cell surface and the amount of ethanol molecules that are catalyzed. Fuel cells can become easily saturated, which makes it problematic to perform multiple measurements within a short time period. Breath analyzers of the sort used by police officers generally require 15 minutes or more between samples to generate accurate readings. Intox, PAS, and other manufacturers of alcohol sensing devices have developed methods to attempt to correlate the breath alcohol concentration to the fuel cell current, including peak current detection and current integration.

In many contexts, it would be desirable to have a breath testing device that could facilitate multiple users in a relatively brief period of time. For example, interest has been mounting in the possibility of a breath testing device designed as a vending machine, which could then be provided to bar or restaurant customers to help them determine their BAC and enable an informed decision as to their own intoxication level. Such a device, of course, would need to be able to provide multiple readings in a row, without the 15-minute wait necessary with commonly used testing devices.

What is needed, therefore, is an apparatus and/or method that can provide for alcohol breath testing for multiple breath samples in a relatively short period of time.

SUMMARY OF THE INVENTION

The present invention accordingly seeks to provide efficient multiple-use alcohol breath testing means, including a vending apparatus incorporating a novel method and apparatus for achieving same.

According to a first aspect of the present invention, then, there is provided an alcohol content determination apparatus comprising air sample vacuum exhaust means.

According to a second aspect of the present invention there is provided a method for determining alcohol content in a breath sample comprising the step of employing vacuum means to exhaust the breath sample.

According to a third aspect of the present invention there is provided an alcohol content determination apparatus comprising resistance control means.

According to a fourth aspect of the present invention there is provided a method for determining alcohol content in a breath sample comprising the step of controlling resistance.

According to a fifth aspect of the present invention there is provided an alcohol content determination apparatus comprising means for calculating a correction factor based on stored breath sample measurements.

According to a sixth aspect of the present invention there is provided a method for determining alcohol content in a breath sample comprising the step of calculating a correction factor based on stored breath sample measurements.

A detailed description of an exemplary embodiment of the present invention is given in the following. It is to be understood, however, that the invention is not to be construed as limited to this embodiment.

## BRIEF DESCRIPTION OF THE DRAWINGS

In the accompanying drawings, which illustrate an exemplary embodiment of the present invention:

**Figure 1** is a simplified view of a prior art fuel cell;

**Figure 2a** is a perspective view of a vending apparatus according to the present invention;

**Figure 2b** is a front elevation view of the vending apparatus of Figure 2a;

**Figure 2c** is a top plan view of the vending apparatus of Figure 2a;

**Figure 2d** is a side elevation view of the vending apparatus of Figure 2a;

**Figure 3a** is a perspective view of the vending apparatus of Figure 2a with front panel text and design features;

**Figure 3b** is a front elevation view of the vending apparatus of Figure 3a;

**Figure 4a** is a partially cut-away perspective view of the vending apparatus illustrating interior components;

**Figure 4b** is a partially cut-away rear elevation view of the vending apparatus illustrating interior components; and

**Figure 4c** is a partially cut-away perspective view of the vending apparatus illustrating interior components.

## DETAILED DESCRIPTION OF EXEMPLARY EMBODIMENT

Referring now in detail to the accompanying drawings, there is illustrated an exemplary embodiment of a method and apparatus according to the present invention.

### *METHOD*

Three novel methods are described below, which each address the problem of fuel cell saturation. They can be practiced separately or in combination, but an exemplary method is presented as a combination of all three methods.
Method 1: It was first determined that the lower the resistance in the external electrical circuit connecting the contacts of the fuel cell, the more current flows and the less the fuel cell saturates with successive breath alcohol samples. To address this determination, the fuel cell output is accordingly shorted in the present invention through a relay at all times that measurement is not being performed (which reduces resistance to very close to 0 ohms) and is put through a 5 ohm resistor when measurement is being performed. While fuel cell manufacturers generally recommend 390 ohms, this would result in a very rapid saturation and a substantial amount of time to clear a saturated condition. The low 5 ohm resistance requires the voltage across the resistance to be amplified by a factor of 100 - 300, which is accomplished in the exemplary embodiment by a differential operational amplifier.
Method 2: It has also been determined that the amount of alcohol in a saturated fuel cell decreases over time as the fuel cell rids itself of ions and the uncatalyzed alcohol is desorbed at the surface back into the surrounding air. To minimize the problem of saturation, then, a vacuum pump is used in this second novel method of the present invention to remove excess breath (which may contain alcohol) as soon as the fuel cell measurement is complete.
Method 3: Finally, it has also been determined that the electrical current produced by the fuel cell is related to not only the-concentration of alcohol at the surface but also to the current saturation state of the fuel cell (that is, the fuel cell has a time-dependent history). According to a third novel method, then, the electrical current produced by the fuel cell is determined for a known alcohol concentration by performing measurements on successive samples and storing the result for a given fuel cell and fuel cell configuration (supporting components including pneumatic tubing, fuel cell temperature, etc.) until a saturated condition is obtained (at which point measurement of successive samples will yield the same fuel cell current). Subsequent periodic measurements of fuel cell current can then be used to determine how the current changes with saturation level. Using the data generated by this third novel method (which may be in the form of a curve or table), a proportional correction factor for the fuel cell may be determined for any degree of saturation.
In practical use, the degree of saturation may be estimated by keeping track of the estimated fuel cell saturation through software. With each breath sample, the estimated degree of saturation is increased in an amount that is proportional to the breath alcohol concentration. After each breath sample, the estimated saturation is decreased with time based on the data collected earlier. At some time after the last breath sample, the estimated fuel cell saturation will reach zero at which point the fuel cell is assumed to be completely unsaturated. It is proper to note that the alcohol saturation and desorption curves are specific to each fuel cell, its configuration, installation, and operating temperature.

Exemplary Method: As mentioned above, while the three novel methods could be worked independently and provide advantages, the exemplary method described below incorporates all three. In addition, a simplified version of the third method above was found to have utility: rather than keeping track of an estimated saturation level and using that to determine the fuel cell proportional constant to use with the measured fuel cell current, the time since last breath sample was used to determine which of three fuel cell calibration values to use. Using this modified third method, this preferred method is as follows:

a. The fuel cell is maintained at a constant temperature of 40°C (104°F) using a closed-loop feedback mechanism;

b. the following sequence is used for each sample (fuel cell output is shorted unless otherwise indicated);

c. measure fuel cell current with output shorted (should be zero);

d. vacuum pump on for 5 seconds to purge lines and fuel cell;

e. vacuum pump off and put fuel cell output across 5 ohm resistor and wait 5 seconds;

f. measure fuel cell current and use this as the zero value (value when no alcohol present);

g. short fuel cell output;

h. if the fuel cell current with output open is not close to the fuel cell current with output shorted, then alcohol must be present, repeat entire process until no alcohol is present;

i. prompt userto blow a long, steady breath sample for 10 seconds;

j. ignore first 5 seconds, used to allow time to obtain deep lung breath sample;

k. turn on vacuum pump and put fuel cell output across 5 ohm resistor;

l. for 5 seconds periodically measure fuel cell current (every 100 milliseconds, for example) and store results;

m. short fuel cell output;

n. calculate blood alcohol content; and

o. leave vacuum on for 45 seconds to purge system.

The blood alcohol content (BAC) is calculated by determining a single statistic or metric V from the 50 sampled data points (presently use average fuel cell current, but can also use peak fuel cell current, integrated fuel cell current, or other statistic). The determined statistic value V is divided by the relevant calibration value $C_i$ and multiplied by the BAC used for calibration (BACCAL) to determine the BAC for the sample:

$$BAC = \frac{V}{C_i} \, BACCAL$$

Three calibration samples (generated by a wet bath simulator with BAC of 0.10 gms%) are measured using the above sequence and these values are stored ($C_i$; i=1, 2, 3) where $C_i$ is the calibration value at index. The calibration samples are spaced approx. 1:40 apart in time and start with a completely unsaturated fuel cell. Once the calibration samples have been stored, new samples can be analyzed and the BAC determined based on the calibration value. The index of the calibration value is used in place of the degree of saturation described earlier as it is much simpler to implement.

When a new sample is taken, the calibration index i for the next sample is empirically estimated:

■ i is increased by 1 for each sample taken where the estimated BAC is > 0.05 gms% (assumes that this increases fuel cell saturation)

■ i is decreased by 1 for each sample taken when the estimated BAC is < 0.025 gms% (assumes that low alcohol breath samples help to clear the fuel cell by promoting desorption of alcohol at the fuel cell surface)

■ *i* is decreased by 1 for each 240 seconds since last sample (assumes that fuel cell saturation has now decreased)

■ *i* is set to zero when 900 seconds elapse since last sample (assumes that fuel cell is now completely unsaturated)

■ When *i* is >3, then a value of 3 is used to determine the calibration value (it has been found that after three samples, the fuel cell is essentially saturated)

■ *i* cannot decrease below 1 (completely unsaturated)

As can be seen, each of the three methods could be practiced independently, although a combination of all three would clearly provide greater advantages. An apparatus for use with the above methods is described in the following.

**APPARATUS**

While various apparatus could be used to practice the above methods, there is a clear need for a vending machine apparatus that can employ the above methods to provide efficient multiple-use alcohol breath testing. The following exemplary apparatus is therefore in the form of a vending machine, although it will be clear to one skilled in the art that other apparatus could be used to work the above methods.

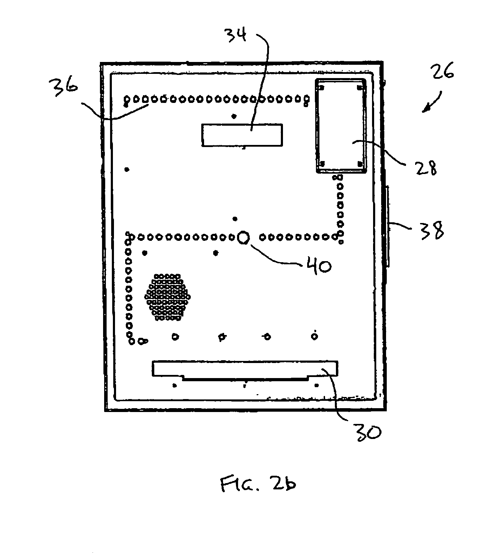

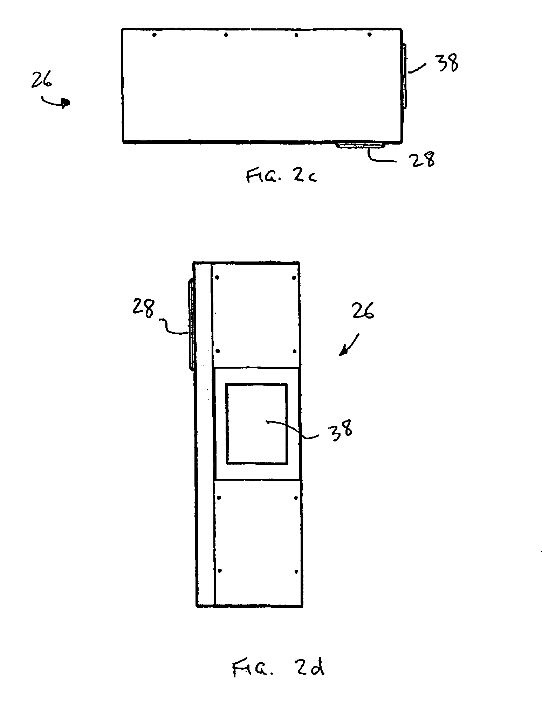

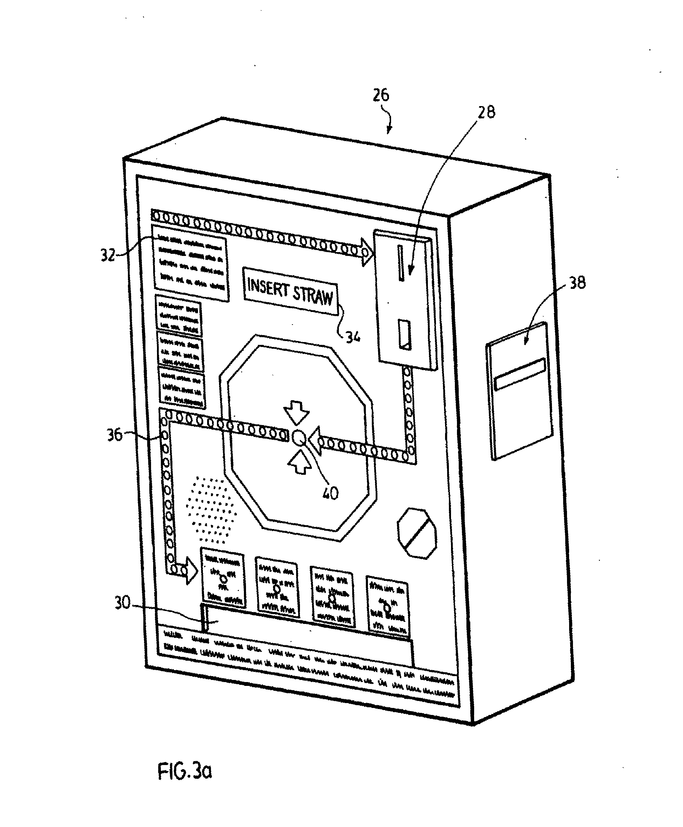

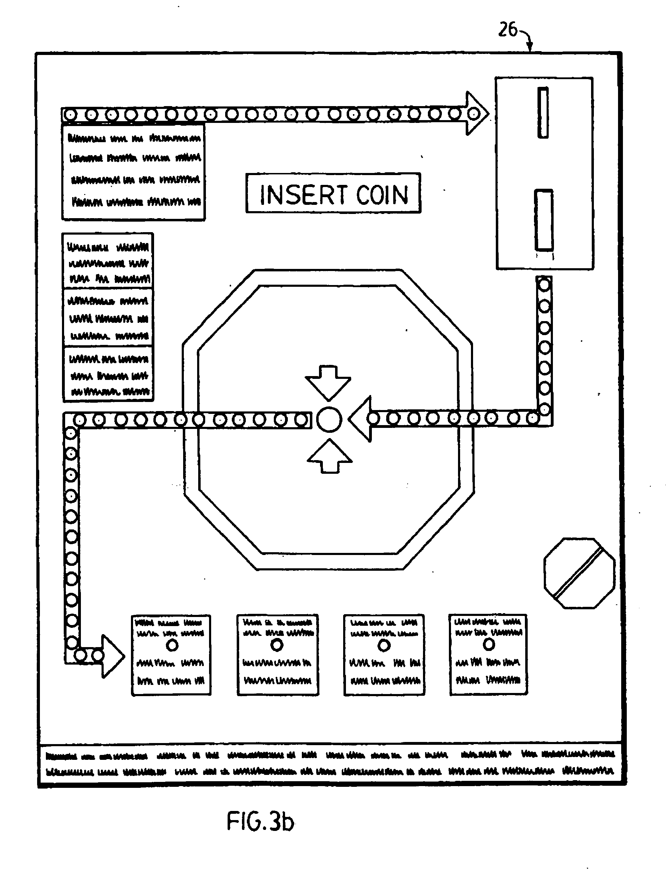

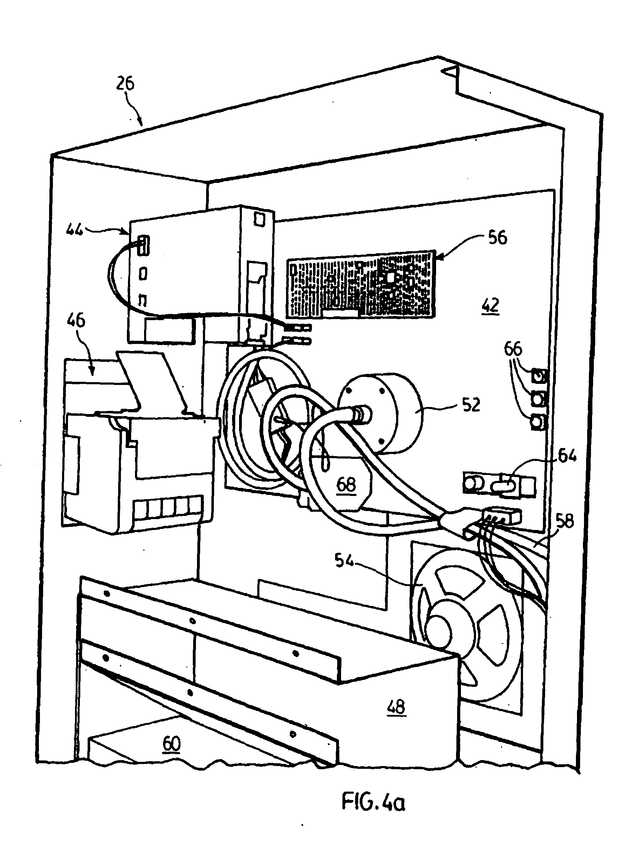

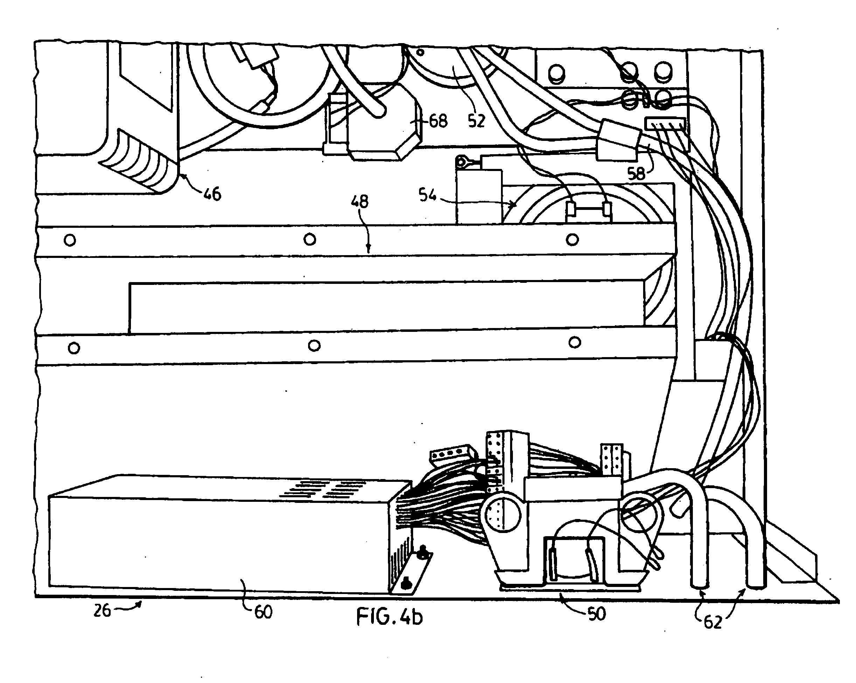

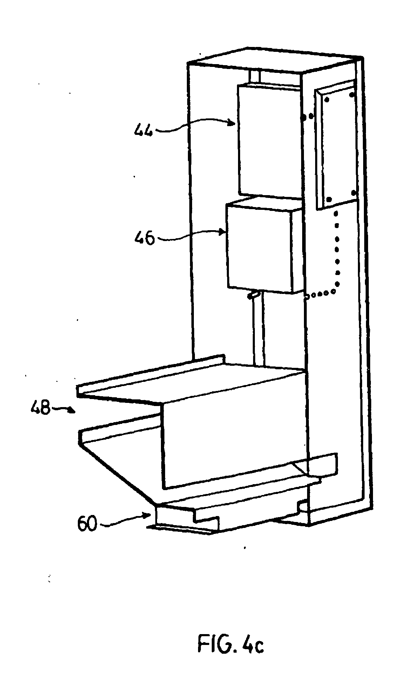

Referring to Figures 2a to 2d, 3a and 3b, there is illustrated the exterior of a vending machine 26 according to the present invention. The front of the vending machine 26 contains the coin acceptor 28, the straw dispenser 30, the test instructions 32, the text display 34, indicator lights 36, optional bill acceptor 38, and the straw hole 40 into which a breath sample is blown. Referring to Figures 4a to 4c, there are illustrated the interior components of the vending machine 26. Inside the vending machine 26 is a printed circuit board 42, a pressure switch 68 (used to detect breath pressure), the coin mechanism 44, optional bill mechanism 46, straw dispenser mechanism 48, coin/bill hopper, pneumatic tubing 58 (connecting the straw sample hole, fuel cell, vacuum pump and exhaust vents), and vacuum pump 50. All access to the inside of the unit is through a locking, hinged left panel. The large, two-part printed circuit board 42 contains the fuel cell alcohol sensor 52, the speaker 54 and volume control 64, the display 56, and all of the electronic circuitry. Below the straw dispenser mechanism 48 are the power supply 60, vacuum pump 50, and exhaust ports 62.

As it is desirable to have a vending machine 26 that is capable of being wall-mounted while containing many internal mechanisms and is also difficult to vandalize, the machine 26 is composed of 16 gauge steel using pressed studs (no external screws) and all access is from the left hand side through a hinged, locking panel. The overall size of the unit is 384 wide x 464 high x 152 mm deep.

Specifications for an exemplary vending machine 26 according to the present invention are set out in the following:

<u>Specifications:</u>

| | |
|---|---|
| **Physical:** | |
| Size (WxHxD) | 384 x 464 x 152 mm |
| Mass | 20 kg |
| Mounting | Wall mount |
| **Environmental:** | |
| Operating Temperature | 0°C to +40°C |
| Storage Temperature | -20°C to +60°C |
| Relative Humidity | 85% max, non-condensing |

(continued)

| Electrical: | |
|---|---|
| Power Input | 120 VAC, 60 Hz (200 watts) |
| | 230 VAC, 50 Hz (200 watts) |
| Power Supply Approvals | CSA 22.2 NO. 60950-00 |
| | IEC 950, UL 1950, CE |
| **Breath Alcohol Sensor:** | |
| Type | Fuel cell (platinum) |
| Accuracy | High analytical specificity to ethanol |
| | Linear response |
| Temperature Control | Closed-loop heater |
| Calibration Frequency | 6 months |
| Sensor Life | 3 years typical |
| **Front Panel:** | |
| Indicator Lights | Sequencing between steps |
| | Final results |
| Multi-function Display | Bright with wide viewing angle |
| Speaker | Voice promoting [promoting?] |
| Straw Holder | Internal (100 straws) |
| Coin Acceptor | Multi-coin with reject |
| **Multi-Function Display:** | |
| Type | Vacuum fluorescent |
| Functions | Text prompting Numerical test results of BAC Scrolling text messages |
| **Coin Acceptor:** | |
| Type | Multi-coin with reject |
| Types of Coins | Up to three (3) different coin types |
| Programming | Pre-programmed at factory |
| Location | Front |
| **Bill Acceptor (Optional):** | |
| Type | Stackerless |
| Bill Insertion | 4-way |
| Programming | Pre-programmed at factory |
| Location | Right side |
| **Enclosure:** | |
| Extreme Dimensions | 384 x 464 x 152 mm |
| Material | Cold-rolled steel, 16 ga. |
| Paint | Black, powder coat |
| Access | All access from hinged, left side |

The multifunction display is used in two modes: to prompt the user through the test sequence and to display results, and to display parameters such as number of coins collected, coin value, test value, etc. Three small pushbutton keys 66 on the left side of the unit interior are used to scroll through parameters and set/change values. When the unit 26 is assembled and the rear hinged left panel is open, these keys 66 are accessible to the user through the left side and can be easily manipulated with the fingers of the left hand, allowing changes to the machine 26 configuration (recalibration, reset coin/bill counters, change test credit value, play, record audio messages). With the hinged left panel closed and locked, the keys 66 are inaccessible and no changes to the machine 26 configuration can be made.

In addition to using the multi-function display to prompt users through the test sequence, audio messages are also used. These messages can be recorded or played through the above pushbutton interface when a special mode is entered on power-up (used to avoid accidentally recording over existing messages).

The front panel incorporates instructions and graphic symbols that prompt the user through the process of inserting money into the machine, inserting a straw into the straw hole, blowing the breath sample, and obtaining the results. The static graphics 32 are complimented by a series of discrete LED indicator lights 36 that flash in sequence to prompt the user to the next step. The static graphics 32 are also complimented by a bit-mapped, multi-function vacuum fluorescent display 34 that is used to display prompts and results. The display 34 shows short messages within the boundary of the screen and longer messages by horizontal scrolling of the message from right to left.

To initialize the vending machine 26, a user plugs the unit 26 into an AC wall outlet and allows a few minutes to heat up and stabilize the temperature of the internal fuel cell breath alcohol sensor 52.

A user interface by means of the key switches 66 allows changes to configuration parameters such as the cost of a breath sample test, the legal limit for blood alcohol concentration (BAC), and the coin counter. The configuration parameters are stored in non-volatile memory and the vending machine 26 retains their values when the unit is unplugged. Changes to the configuration parameters can only be made by accessing the three push-button switches 66 located inside the unit on the left side. To change any configuration parameters, the user will interact with the three switches 66 in a manner dictated by the instructions and obvious to one skilled in the art.

**Configuration Parameters:**

*Coin Count:* The number of coins is counted and can be displayed with this configuration parameter. Changing the value will reset the coin counter to zero.

*Bill Count:* The number of bills is counted and can be displayed with this configuration parameter. Changing the value will reset the bill counter to zero. This configuration parameter has no meaning and can be ignored, if a bill acceptor is not installed.

*Test Credit:* The cost of a single breath alcohol sample test can be adjusted with this configuration parameter.

*Coin Credit:* The base credit for a single coin can be adjusted with this configuration parameter. It has been preset at the factory to an appropriate value for the coin acceptor.

*Bill Credit:* The base credit for a single bill can be adjusted with this configuration parameter. It has been preset at the factory to an appropriate value for the bill acceptor (if one is installed).

*Version:* The version of software installed in the vending machine 26 is shown with this configuration parameter. It cannot be adjusted.

*Serial Number.* The serial number of the vending machine 26 is shown with this configuration parameter. It cannot be adjusted.

*Calibrate?:* Periodically, recalibration of the fuel cell is required. This configuration parameter starts a recalibration sequence when the value is changed to YES. Once the calibration has been performed, the CALIBRATE OK? prompt is displayed and must be changed to YES to accept the new calibration values.

*Legal Limit:* Different jurisdictions have different legal limits for BAC, so this limit can also be adjusted.

While a particular embodiment of the present invention has been described in the foregoing, it is to be understood that other embodiments are possible within the scope of the invention and are intended to be included herein. It will be clear to any person skilled in the art that modifications of and adjustments to this invention, not shown, are possible without departing from the spirit of the invention as demonstrated through the exemplary embodiment. The invention is therefore to be considered limited solely by the scope of the appended claims.

**Claims**

1. An apparatus for determining alcohol content in an air sample, the apparatus comprising:

   air sample input means for allowing the air sample to enter a location adjacent an electrochemical device;
   the electrochemical device configured to sense the alcohol content in the air sample; and
   air sample exhaust means for removing the air sample from the location adjacent the electrochemical device.

2. The apparatus of Claim 1 wherein the air sample exhaust means comprise a vacuum pump.

3. The apparatus of Claim 1 wherein the electrochemical device comprises a fuel cell, the fuel cell configured to generate electrical current proportional to the alcohol content in the air sample.

4. The apparatus of Claim 1 wherein the air sample input means comprise a tube for injecting the air sample into the apparatus.

5. The apparatus of Claim 1 further comprising tubing connecting the air sample input means and the electrochemical device, and connecting the electrochemical device and the air sample exhaust means.

6. An apparatus for determining alcohol content in an air sample, the apparatus comprising:

air sample input means for allowing the air sample to enter a location adjacent an electrochemical device;
the electrochemical device for sensing the alcohol content in the air sample;
resistance control means for selectively lowering resistance across the electrochemical device to generate a lower resistance, to enable increased current flow through the electrochemical device;
voltage amplification means for increasing voltage across the lower resistance; and
air sample output means.

7. The apparatus of Claim 6 wherein the electrochemical device comprises a fuel cell, the fuel cell configured to generate electrical current proportional to the alcohol content in the air sample.

8. The apparatus of Claim 6 wherein the air sample input means comprise a tube for injecting the air sample into the apparatus.

9. The apparatus of Claim 6 wherein the resistance control means comprises a 5 ohm resistor.

10. The apparatus of Claim 6 wherein the voltage amplification means comprise a differential operational amplifier.

11. An apparatus for determining alcohol content in an air sample, the apparatus comprising:

air sample input means for allowing the air sample to enter a location adjacent an electrochemical device;
the electrochemical device for sensing an uncorrected alcohol content in the air sample;
measurement data storage means for storing measurement data corresponding to air sample readings at various electrochemical device saturation levels;
correction factor calculation means for using the measurement data from the measurement data storage means to correct the uncorrected alcohol content; and
air sample output means.

12. The apparatus of Claim 11 wherein the air sample input means comprise a tube for injecting the air sample into the apparatus.

13. The apparatus of Claim 11 wherein the measurement data comprises electrical current data for known alcohol concentrations at various saturation levels for the electrochemical device.

14. The apparatus of Claim 11 wherein the correction factor calculation means uses a series of calibration values based on time since last air sample measurement.

15. An apparatus for determining alcohol content in an air sample, the apparatus comprising:

air sample input means for allowing the air sample to enter a location adjacent an electrochemical device;
the electrochemical device configured to sense an uncorrected alcohol content in the air sample;
measurement data storage means for storing measurement data corresponding to air sample readings at various electrochemical device saturation levels;
correction factor calculation means for using the measurement data from the measurement data storage means to correct the uncorrected alcohol content;
resistance control means for selectively lowering resistance across the electrochemical device to generate a lower resistance, to enable increased current flow through the electrochemical device;
voltage amplification means for increasing voltage across the lower resistance; and
air sample exhaust means for removing the air sample from the location adjacent the electrochemical device.

16. A method for determining alcohol content in an air sample, comprising the steps of:

a. providing an apparatus comprising an electrochemical device configured to sense the alcohol content in the air sample;
b. injecting the air sample to a location adjacent the electrochemical device;
c. allowing the air sample to contact the electrochemical device for a predetermined period; and
d. ejecting the air sample from the location adjacent the electrochemical device at expiry of the predetermined period of time to reduce saturation of the electrochemical device.

**17.** A method for determining alcohol content in an air sample, comprising the steps of:

a. providing an apparatus comprising: an electrochemical device configured to sense the alcohol content in the air sample; resistance control means for selectively lowering resistance across the electrochemical device to generate a lower resistance, to enable increased current flow through the electrochemical device; and voltage amplification means for increasing voltage across the lower resistance;
b. injecting the air sample to a location adjacent the electrochemical device;
c. allowing the air sample to contact the electrochemical device to generate an electrochemical device current output;
d. allowing the electrochemical device current output to pass through the resistance control means;
e. amplifying the voltage across the lower resistance; and
f. allowing the air sample to move from the location adjacent the electrochemical device.

**18.** A method for determining alcohol content in an air sample, comprising the steps of:

a. providing an apparatus comprising: an electrochemical device for sensing an uncorrected alcohol content in an air sample; measurement data storage means for storing measurement data corresponding to air sample readings at various electrochemical device saturation levels; and correction factor calculation means for using the measurement data from the measurement data storage means to correct the uncorrected alcohol content;
b. determining measurement data corresponding to air sample readings at various electrochemical device saturation levels;
c. storing the measurement data in the measurement data storage means;
d. injecting the air sample to a location adjacent the electrochemical device;
e. allowing the air sample to contact the electrochemical device to enable determination of an uncorrected alcohol content value;
f. using the correction factor calculation means to calculate a correction factor based on the measurement data;
g. applying the correction factor to the uncorrected alcohol content value to arrive at a corrected alcohol content value; and
h. allowing the air sample to move from the location adjacent the electrochemical device.

**19.** A method for determining alcohol content in an air sample, comprising the steps of:

a. providing an apparatus comprising: an electrochemical device for sensing an uncorrected alcohol content in an air sample; calibration data storage means for storing calibration data corresponding to time since last air sample measurement; and correction factor calculation means for using the calibration data from the calibration data storage means to correct the uncorrected alcohol content;
b. determining calibration data for the electrochemical device;
c. storing the calibration data in the calibration data storage means;
d. injecting the air sample to a location adjacent the electrochemical device;
e. allowing the air sample to contact the electrochemical device to enable determination of an uncorrected alcohol content value;
f. using the correction factor calculation means to calculate a correction factor based on the calibration data;
g. applying the correction factor to the uncorrected alcohol content value to arrive at a corrected alcohol content value; and
h. allowing the air sample to move from the location adjacent the electrochemical device.

**20.** A method for determining alcohol content in an air sample, comprising the steps of:

a. providing an apparatus comprising: an electrochemical device for sensing an uncorrected alcohol content in an air sample; resistance control means for selectively lowering resistance across the electrochemical device to generate a lower resistance, to enable increased current flow through the electrochemical device; voltage amplification means for increasing voltage across the lower resistance; calibration data storage means for storing calibration data corresponding to time since last air sample measurement; correction factor calculation means for using the calibration data from the calibration data storage means to correct the uncorrected alcohol content; and air sample exhaust means for removing the air sample from the location adjacent the electrochemical device;
b. determining calibration data for the electrochemical device;
c. storing the calibration data in the calibration data storage means;

d. shorting current output from the electrochemical device;

e. purging any previous air samples using the air sample exhaust means;

f. allowing the current output across the resistance control means to arrive at an alcohol-free zero value;

g. shorting current output from the electrochemical device;

h. injecting the air sample to a location adjacent the electrochemical device;

i. allowing the air sample to contact the electrochemical device to generate an electrochemical device current output to allow determination of an uncorrected alcohol content value;

j. allowing the electrochemical device current output to pass through the resistance control means;

k. amplifying the voltage across the lower resistance;

l. shorting current output from the electrochemical device;

m. using the correction factor calculation means to calculate a correction factor based on the calibration data;

n. applying the correction factor to the uncorrected alcohol content value to arrive at a corrected alcohol content value; and

o. purging the air sample using the air sample exhaust means.

PRIOR ART

FIG. 1

FIG. 2a

FIG. 2b

Fig. 2c

Fig. 2d

INSERT STRAW

FIG.3a

26

INSERT COIN

FIG.3b

FIG.4a

FIG.4b

EP 1 867 987 A2

FIG. 4c